# FASCICULE DE BREVET EUROPEEN

(11) **EP 1 113 767 B1**
(45) Date de publication et mention de la délivrance du brevet: **17.11.2004**
(21) Numéro de dépôt: 99942969.9
(22) Date de dépôt: 14.09.1999
(51) Int. Cl.: A61F 5/00, A61B 17/12

(54) **DISPOSITIF DE CONSTRICTION GASTRIQUE**
MAGENBAND
GASTRIC CONSTRICTION DEVICE

(30) Priorité: 14.09.1998 FR 9811592
(43) Date de publication de la demande: 11.07.2001
(73) Titulaire: SOFRADIM PRODUCTION, 01600 Trévoux (FR)
(72) Inventeur: DARGENT, Jérôme, F-69002 Lyon (FR); GREILLIER, Bernard, F-69008 Lyon (FR)
(74) Mandataire: Guerre, Dominique
(86) Numéro de dépôt international: PCT/FR1999/002186
(87) Numéro de publication internationale: WO 2000/015158

(56) Documents cités:
- EP-A- 0 876 808
- WO-A-96/01597
- DE-A- 1 541 262
- DE-A- 19 718 903
- DE-A- 19 751 733
- FR-A- 2 730 406
- GB-A- 1 174 814
- US-A- 3 840 018
- US-A- 4 118 805
- US-A- 5 601 604

## Description

La présente invention concerne un dispositif de constriction, au moins en partie implantable dans le corps humain ou animal. L'invention sera plus particulièrement décrite par rapport à un dispositif de constriction gastrique du type "anneau de gastroplastie", pouvant être utilisé pour le traitement de l'obésité morbide.

L'invention ne se limite pas cependant à une telle application ou utilisation. L'invention trouve également son utilité dans diverses interventions médicales sur l'homme ou sur l'animal, dans lesquelles il est nécessaire d'obtenir provisoirement ou de façon permanente, une constriction, le cas échéant une occlusion d'un organe ou d'un conduit ou canal.

Dans le domaine de la constriction gastrique, plusieurs raisons existent de vouloir contrôler le débit alimentaire gastrique d'une personne, et notamment, mais pas exclusivement, dans le cas d'une personne souffrant d'une obésité morbide, ou par exemple en cas de diabète, hypercholestérolémie, ou encore d'hypertension artérielle. Par l'expression "l'obésité morbide", on entend le fait qu'une personne a une surcharge pondérale d'au moins 45 kg, ou bien au moins égale à son poids idéal, tel que l'on peut l'établir en fonction de sa taille, et au moyen d'une table de calcul de l'index de masse corporelle.

Plusieurs techniques chirurgicales sont connues pour essayer de traiter cette obésité, par exemple en raccordant l'estomac directement sur l'intestin grêle. Une autre technique consiste à placer dans la paroi de l'estomac des sutures ou des agrafes, verticalement ou horizontalement, afin de définir une poche de taille plus réduite que celle de l'estomac, ce qui limite la quantité et la vitesse de passage des aliments pouvant être ingérés. Toutefois, et bien que ces solutions antérieures soient relativement efficaces, elles sont assez traumatisantes, et peu ou pas réversibles, et leur faisabilité par coelioscopie est assez aléatoire.

Une autre solution proposée a été celle de disposer un ballon gonflable à l'intérieur de l'estomac, ce qui donne au malade l'impression constante de satiété. Un tel dispositif a été décrit dans le brevet US-C-4 416 267. Cependant, la pose de ballons de ce type a conduit à un nombre de complications non négligeables, telles que blocages intestinaux, ulcères gastriques, et ne prend pas en compte le risque du patient associé à un contact prolongé d'un objet étranger avec les muqueuses gastriques.

Enfin, une approche beaucoup moins traumatisante est celle dans laquelle on utilise un dispositif de constriction gastrique, comprenant premièrement un organe de constriction gastrique formant dans sa configuration opératoire un anneau, par exemple comprenant une bande flexible, dont les deux extrémités sont adjacentes l'une par rapport à l'autre dans la configuration opératoire, munie d'un ballonnet gonflable, entourant la paroi extérieure de l'estomac, deuxièmement un moyen d'actionnement de l'organe de constriction gastrique, par exemple une valve ou injecteur de fluide pour injecter ou introduire ledit fluide dans le ballonnet, et troisièmement un moyen de commande à distance du moyen d'actionnement, par exemple une chambre d'introduction de fluide, située juste en dessous de la peau à un endroit relativement facile d'accès pour le malade. Le moyen de commande et le moyen d'actionnement sont reliés par un conduit flexible, tel qu'un cathéter, permettant de transmettre le fluide depuis la chambre vers le moyen d'actionnement. Un tel dispositif présente l'avantage qu'il peut être introduit dans le corps par coelioscopie ou laparoscopie, ce qui réduit de manière significative les traumatismes et complications post-opératoires associés à l'intervention chirurgicale, par rapport à la voie dite "ouverte", c'est-à-dire la chirurgie abdominale classique ; mais un tel dispositif présente le désavantage que la chambre est assez encombrante à long terme. Un dispositif correspondant à la description précédente a été développé, et est connu par exemple, par les travaux du Docteur Kuzmak, et on se référera utilement aux brevets ou aux demandes de brevets WO-A-92/02182, US-A-4 592 339, EP-A-O 611 561, WO-A-86/04498, WO-A-94/27504, et US-A-5 226 429.

Toutefois, aucune des solutions proposées jusqu'à présent ne permet de contrôler avec précision le degré de constriction gastrique. Dans le cas des dispositifs du Docteur Kuzmak, les volumes de fluide injectable sont limités, et représentent une solution qui est pour le moins difficile à contrôler de manière précise. Il est possible d'injecter dans, ou retirer du ballonnet un certain volume de fluide, mais l'élasticité intrinsèque de ce dernier varie en fonction des conditions biologiques de son implantation, et avec le temps, ce qui rend son expansion difficilement contrôlable, et insuffisamment adaptée au traitement souhaité ou désirable. Par ailleurs, l'intervention manuelle, par exemple l'appui par le patient sur le bouton poussoir sous-cutané, voire même le déplacement accidentel de ce dernier peuvent avoir des conséquences indésirables, par exemple la nécessité de réintervention, ou de désinfection du matériel etc.

Il est connu de réaliser un dispositif de constriction, au moins en partie implantable dans le corps humain ou animal, comprenant un organe de constriction formant dans sa configuration opératoire un anneau, ledit organe de constriction comportant une bande flexible, dont les deux extrémités sont adjacentes l'une par rapport à l'autre dans la configuration opératoire, ainsi qu'un moyen d'actionnement de l'organe de constriction.

On connaît ainsi, par l'intermédiaire du document EP-0 876 808, un dispositif de constriction gastrique à bande flexible, implantée et fermée sur elle-même autour de l'estomac.

La bande flexible comporte dans sa configuration opératoire, sur sa face intérieure, une paroi souple délimitant une cavité remplie d'un volume variable de liquide provenant d'un réservoir de compensation, également implanté dans le corps du patient.

Une pompe télécommandée peut transmettre le liquide du réservoir de compensation vers la bande gastrique, et inversement, par l'intermédiaire d'une unité électrique de commande. Cette dernière est montée avec la pompe dans une boîte de commande implantée sous la peau du patient.

Outre les inconvénients déjà précités, concernant ce type de dispositif, il y a des risques liés aux fuites éventuelles de liquide, consécutivement à une mauvaise manipulation, ou à un défaut de fabrication.

Par ailleurs, le gonflage de la bande gastrique favorise l'apparition de plis sur sa paroi intérieure, en contact avec l'estomac. Une constriction uniforme n'est donc plus appliquée sur la périphérie de l'estomac, et le risque de coincer la paroi de l'estomac dans un pli augmente.

La pompe est alimentée par une source d'énergie électrique, implantée dans le corps, ce qui peut être dangereux, en fonction des matériaux constitutifs d'une telle source, par exemple d'une pile électrique.

En outre, il est connu par le document US-A-3 840 018 un dispositif au moins en partie implantable dans le corps humain ou animal, comprenant un organe de constriction formant anneau et un moyen d'actionnement de cet organe de constriction. L'anneau que forme cet organe de constriction comporte une bande flexible dont au moins une des extrémités comporte un élément tractable ; l'extrémité tractable de la bande flexible est déplacée à l'intérieur d'un élément creux du dispositif, via un organe de traction que comprend le moyen d'actionnement. Cet organe de traction est constitué par une vis sans fin, qui permet de générer une déformation radiale de l'organe de constriction.

Le document DE 197 18 903 décrit, de même que le brevet US-A-3 840 018 ci-dessus, l'ensemble des caractéristiques visées dans le préambule de la revendication 1.

La présente invention prévoit un dispositif de constriction permettant de pallier aux inconvénients des dispositifs de l'état de la technique, et de régler de manière très précise, et reproductible, la constriction appliquée à tout organe ou conduit du corps humain ou animal, tout en évitant ou limitant l'intervention chirurgicale requise pour le réglage.

Selon la présente invention, en coopération, d'une part au moins une extrémité de la bande flexible comporte un élément tractable, permettant de déplacer ladite extrémité par rapport à l'autre extrémité, en générant une déformation radiale de l'organe de constriction, et d'autre part le moyen d'actionnement comprend un organe de traction de l'élément tractable. Selon une réalisation avantageuse de l'invention, l'élément tractable est lamelliforme. Selon une réalisation avantageuse, la bande flexible est fixée par l'une de ces extrémités au corps du moyen d'actionnement, et comporte l'élément tractable du côté de son autre extrémité. Ledit élément tractable comprend des moyens de traction, par exemple des crans, aptes à coopérer avec l'organe de traction du moyen d'actionnement.

Le dispositif de la présente invention est également remarquable par le fait que, dans la configuration prête à ceindre de l'organe de constriction, entre ces deux extrémités, la bande flexible est composée d'un premier élément comportant un premier organe de fixation, par exemple mâle, et d'un deuxième élément comportant un deuxième organe de fixation complémentaire, par exemple femelle, le premier élément étant relié au deuxième élément par accouplement définitif des organes de fixation complémentaires, pour obtenir la configuration opératoire de l'organe de constriction.

Selon une réalisation avantageuse du dispositif de constriction, l'extrémité fixe de la bande flexible est traversée par une lumière longitudinale et débouchante, apte à recevoir une languette de largeur réduite de l'élément tractable. Avantageusement, l'organe de constriction comporte une gaine en matière bio-compatible, compressible ou pliable, renfermant la bande flexible. Cette gaine permet d'assurer une compatibilité biologique de l'organe de constriction avec la paroi extérieure de l'estomac car le dispositif de constriction gastrique doit demeurer en place pendant une période relativement longue, afin d'obtenir un résultat clinique satisfaisant, et il donc préférable de diminuer, autant que possible, les risques de lésion dus au contact d'un corps étranger avec les tissus de la paroi de l'estomac.

Avantageusement, le moyen d'actionnement comprend un moteur bi-directionnel couplé à l'organe de traction.

L'organe de traction, selon l'invention, engrène avec les moyens de traction pour déplacer l'élément tractable selon une direction longitudinale et circonférentielle de l'organe de constriction.

Selon un exemple avantageux de la réalisation, l'organe de traction est une vis tangentielle, tandis que les moyens de traction sont des crans, régulièrement espacés les uns des autres, transversaux par rapport à la direction longitudinale, et le filetage de la vis tangentielle a le même pas que celui séparant les crans de traction.

Avantageusement, le dispositif de constriction comporte une coque protectrice présentant une partie en matière rigide, formant boîtier de moteur, et une partie en matière flexible bio-compatible, compressible ou pliable, formant la gaine de la bande flexible.

Avantageusement, le boîtier et la gaine forment une seule et même pièce.

Avantageusement, la gaine présente des évidements de matière de part et d'autre de la bande flexible, et / ou des plis préformés, permettant à ladite gaine de se plier de manière ordonnée et régulière, lors de la déformation radiale de l'organe de constriction.

Selon un mode réalisation intéressant, le dispositif comprend ou est associé à un moyen de commande à distance. Ce dernier peut comprendre un émetteur extra-corporel et un récepteur intra-corporel, ce dernier comportant un moyen de traitement d'un signal émis par l'émetteur d'une part, et de transmission d'un signal de commande au moyen d'actionnement d'autre part. Le moyen de traitement peut comprendre un seul circuit électrique agencé pour véhiculer à la fois des informations de commande et de l'énergie vers le moyen d'actionnement.

Avantageusement, le moyen de traitement peut comprendre un circuit électrique constitué par une bobine d'induction résonnante, un redresseur, un générateur d'impulsions, au moins un circuit de registre à décalage, et un circuit de commande de la direction de marche du moyen d'actionnement.

A titre d'exemple conforme à l'invention, le récepteur du moyen de commande est disposé à distance de l'organe de constriction, et relié électriquement au moyen d'actionnement.

Selon un autre exemple de réalisation avantageux du dispositif conforme à l'invention, l'émetteur extra-corporel comprend un circuit électrique constitué par une alimentation en énergie, un oscillateur, un circuit amplificateur et une bobine d'induction primaire.

L'émetteur peut également comprendre, en outre, au moins un organe de sélection de fréquence de l'oscillateur.

Le dispositif de constriction conforme à l'invention est également remarquable dans la mesure où le moyen d'actionnement est agencé pour conserver de façon stable la position relative des deux extrémités de la bande flexible, l'une par rapport à l'autre, en l'absence d'apport d'énergie audit moyen d'actionnement.

La présente invention sera mieux comprise par la description détaillée suivante d'un mode d'exécution préféré, accompagnée du dessin en annexe, dans lequel :
- la **Figure 1** est une vue schématique en perspective, partiellement arrachée de l'abdomen d'un corps humain, dans lequel une partie d'un dispositif de constriction gastrique selon l'invention a été implantée ;
- la **Figure 2** est une vue en perspective, partiellement arrachée, de la partie du dispositif de constriction gastrique selon l'invention, implantable dans le corps humain ;
- la **Figure 3** est une vue en coupe de l'organe de constriction gastrique, dans sa configuration opératoire, et du moyen d'actionnement d'un dispositif selon l'invention ; l'organe de constriction est représenté à l'état non déformé, avec une section transversale maximum de l'anneau de constriction.
- la **Figure 4** est une vue en coupe de l'organe de constriction de la **Figure 3**, en cours d'actionnement ; l'anneau de constriction a une section transversale réduite par rapport à celle représentée à la **figure 3**.
- la **Figure 5** est une vue schématique des circuits électriques du moyen de commande à distance d'un dispositif de constriction gastrique selon l'invention.
- la **Figure 6** est une vue schématique plus détaillée d'un des circuits électriques de la Figure 5 ;
- la **Figure 7** est une vue en perspective, développée et à plat de la bande flexible appartenant à l'organe de constriction représenté aux figures 2 à 4.

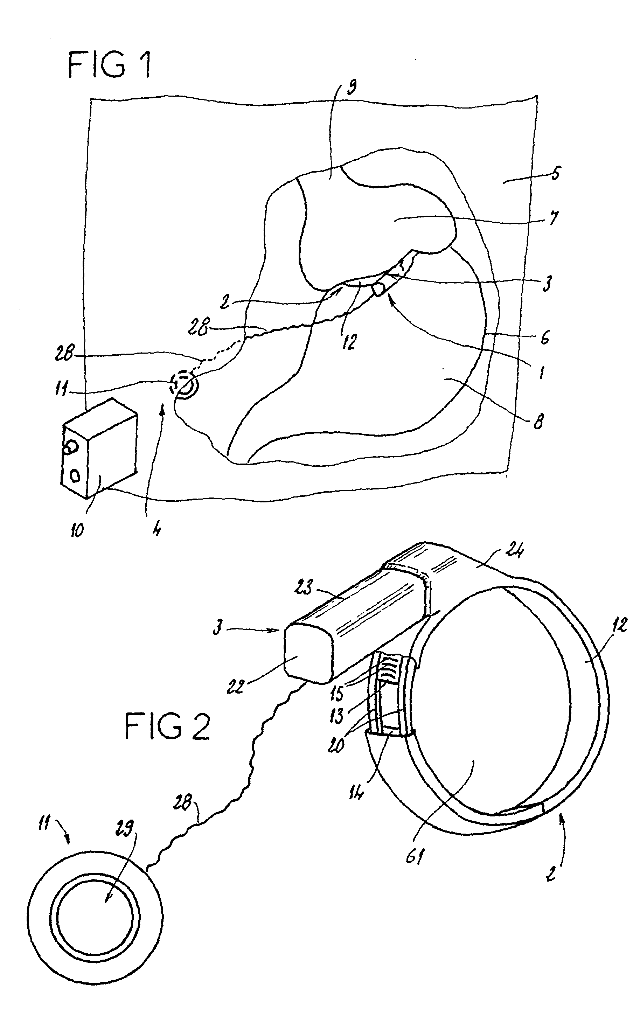

Se référant maintenant à la Figure 1, le dispositif de constriction notamment gastrique selon l'invention est indiqué de manière générale par la référence 1. Ce dispositif comprend un organe 2 de constriction gastrique formant sensiblement un anneau de constriction de section transversale 61, variable, un moyen d'actionnement 3 de l'organe 2 de constriction gastrique, et un moyen de commande 4 à distance du moyen d'actionnement 3.

Le dispositif de constriction gastrique 1 est implanté pour partie dans le corps 5, représenté schématiquement, l'organe de constriction 2 formant anneau entourant la paroi extérieure de l'estomac 6, de manière à créer une poche supérieure 7, de taille réduite, et une poche inférieure 8, de taille plus importante que la poche supérieure. Le passage d'aliments depuis l'oesophage 9 est limité, d'une part par le volume restreint de la poche supérieure 7, et d'autre part par le degré de constriction appliqué par l'organe de constriction 2. Ainsi, et de manière généralement connue, on contrôle le débit alimentaire, par exemple pour faire maigrir une personne atteinte d'obésité morbide.

Le moyen de commande 4 comprend un émetteur 10, et un récepteur 11, le récepteur 11 comportant un moyen de traitement d'un signal émis par l'émetteur 10, et de transmission d'un signal de commande vers le moyen d'actionnement 3, afin de pouvoir régler avec précision le degré de constriction de l'organe de constriction 2. L'émetteur 10 et le récepteur 11, ainsi que les moyens les composant, seront décrits plus en détails ci-après. Le récepteur 11 du moyen de commande 4 est disposé à distance de l'organe 2 de constriction, et relié électriquement, par un fil 28, au moyen d'actionnement 3.

Conformément à la présente invention, et tel que représenté par la Figure 2, l'organe de constriction 2 comprend une bande flexible 12, lamelliforme, par exemple en polyéthylène ou polypropylène, et une gaine 14 en matière flexible biocompatible, compressible ou pliable, par exemple en silicone, renfermant une bande flexible 12. Cette gaine permet d'assurer une réduction des traumatismes subis par la paroi extérieure de l'estomac, lors des mouvements de cette dernière contre l'organe de constriction 2, pendant le passage d'aliments de la poche supérieure 7 vers la poche inférieure 8. Etant donné que le diamètre formé par la bande flexible 12 varie en fonction de la constriction gastrique à appliquer par le biais du moyen d'actionnement 3, il est préférable que la gaine 14 soit constituée d'une matière compressible ou pliable, de façon à ce qu'elle ne gêne pas cette augmentation ou réduction de la section transversale 61 de constriction.

La bande flexible peut avoir toute forme autre que lamelliforme, par exemple une section circulaire.

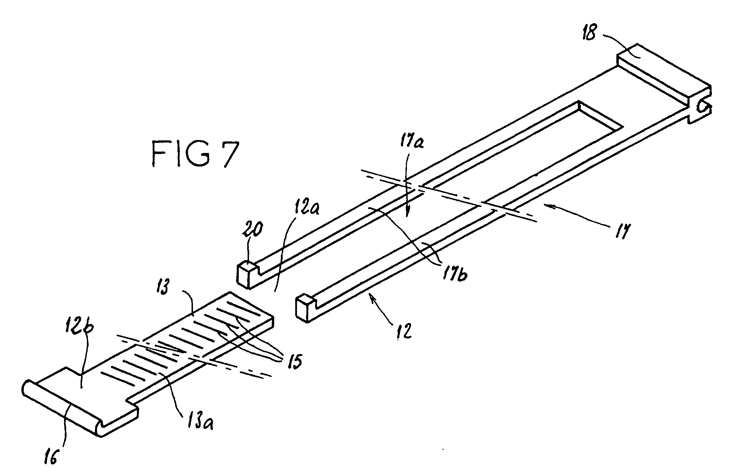

Comme représenté par référence aux figures 2 à 4, dans la configuration opératoire du dispositif de constriction, et comme mieux compris par référence à la figure 7, la bande flexible 12 présente une configuration en forme de boucle, dont les deux extrémités 12a et 12b, ou brins extrêmes, sont l'un (12a) fixe, et l'autre mobile (12b), selon une trajectoire prédéterminée. De manière générale, les deux extrémités 12a et 12b sont et demeurent adjacentes l'une par rapport à l'autre, en se chevauchant ou se superposant selon une trajectoire de mouvement relatif, linéaire mais courbe, par exemple circonférentielle, mais concentriquement l'une par rapport à l'autre, selon le degré de constriction désiré, et ce avec le moyen d'actionnement 3 décrit ci-après.

De manière à déplacer par rapport à l'extrémité fixe 12a, l'extrémité mobile 12b, d'une part celle-ci comporte un élément tractable 13, lui-même lamelliforme, et d'autre part le moyen d'actionnement 3 comprend un organe de traction 25 de l'élément tractable, pour déplacer l'extrémité mobile 12b de la bande flexible 12 par rapport à l'extrémité fixe 12a, en générant une déformation radiale de l'organe de constriction 2, et plus exactement une section transversale 61 de constriction, variable.

L'extrémité 12a de la bande flexible 12 est fixée par un moyen 20 au corps du moyen d'actionnement 3, et l'élément tractable 13, du côté de l'extrémité libre 12b, comprend des moyens de traction, par exemple des crans 15 aptes à coopérer avec l'organe de traction 25.

Comme représenté à la figure 7, l'extrémité fixe 12a de la bande flexible 12 est traversée par une lumière 17a, longitudinale et débouchante, apte à recevoir une languette 13a de largeur réduite de l'élément tractable 13. La longueur développée de la lumière 17a est adaptée à la course de l'extrémité 12b par rapport à l'extrémité 12a, selon la trajectoire de chevauchement, à savoir dans le cas présent, d'insertion de l'extrémité libre 12b par rapport à l'extrémité fixe 12a.

Selon un exemple de réalisation avantageux conforme à l'invention, la gaine 14 peut présenter des propriétés extensibles de manière à améliorer le contact avec l'organe soumis à la constriction. En effet, il est alors possible de partir d'une position initiale sans constriction de la bande flexible 12, dans laquelle la gaine 14 est étirée. Lors de la constriction, la gaine 14 suit alors la forme rétractée de la bande flexible 12 en se contractant.

L'élément 13 à l'extrémité 12b est tractable par l'organe de traction 25, de manière à pouvoir être déplacé dans deux sens longitudinaux circonférentiels opposés, à savoir un sens de réduction de la constriction gastrique, et un sens d'augmentation de la constriction gastrique. Ainsi, l'élément tractable 13 présente de préférence, et comme représenté schématiquement par la Figure 2, des crans de traction 15, disposés, par exemple, sensiblement transversalement à la direction longitudinale de l'élément tractable 13, et régulièrement espacés les uns des autres, selon un pas prédéterminé, et permettant, en coopération avec l'organe de traction 25 consistant en une vis sans fin, de faire déplacer l'élément 13 dans un sens de réduction de la constriction gastrique, ou au contraire, dans un sens opposé. Les crans de traction 15 peuvent prendre d'autres formes, par exemple fentes, évidements ou dents, l'essentiel étant qu'ils puissent coopérer ou engrener avec l'organe de traction 25 correspondant, prévu sur le moyen d'actionnement 3.

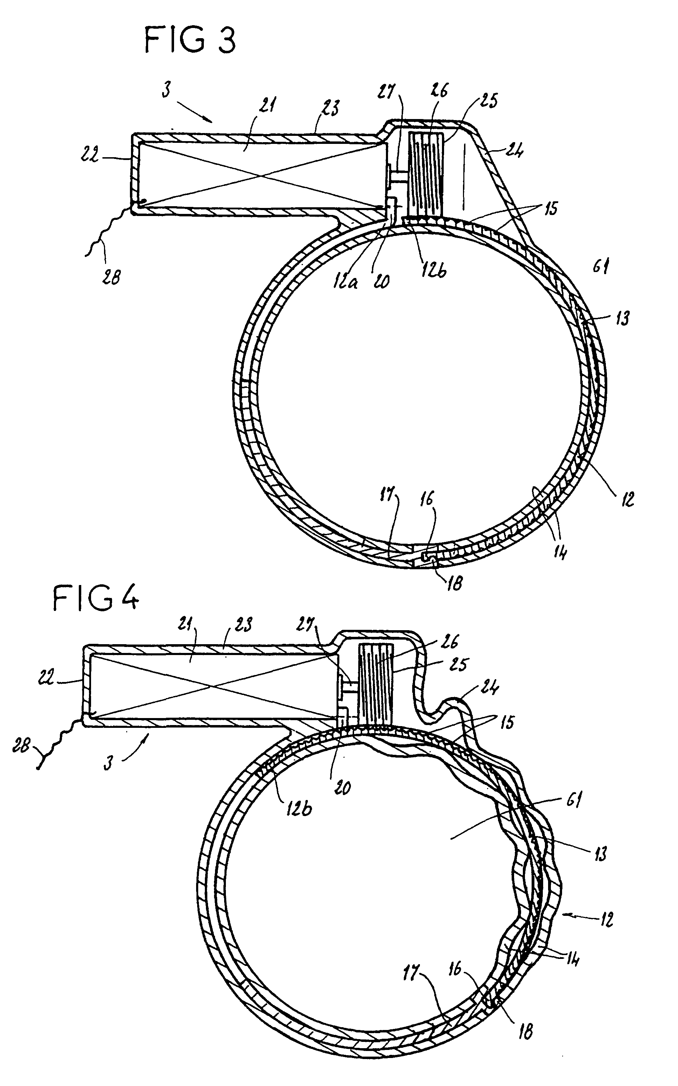

Par ailleurs, selon le mode d'exécution préféré de l'invention, et tel qu'illustré par la Figure 3, dans une configuration prête à ceindre de l'organe 2 de constriction, la bande flexible 12 est composée d'un premier élément 13 comportant un premier organe de fixation 16, par exemple mâle, et d'un deuxième élément 17 comportant un deuxième organe de fixation 18 complémentaire, par exemple femelle, le premier élément 13 étant relié au deuxième élément 17 par accouplement définitif des organes de fixation 16,18 complémentaires. Cette disposition permet notamment de moduler par constriction la longueur de la bande flexible 12, par exemple pour s'adapter à un diamètre de constriction gastrique plus important, ce qui peut être le cas notamment si on désire créer une poche supérieure 7 de volume équivalent à la poche inférieure 8.

La division de la bande flexible 12 en deux éléments ou branches 13 et 17 accouplables facilite grandement la pose de l'organe 2 de constriction autour de l'estomac, puisque dans ce cas les extrémités des éléments 13 et 17 sont accouplées après que ces éléments aient été passés lors de l'intervention chirurgicale autour de l'estomac.

Comme montré aux figures 3, 4 et 7, l'extrémité libre 12a de la bande flexible 12, correspondant au deuxième élément 17, est traversée, sur la plus grande partie de sa longueur et à l'opposé de son extrémité portant l'organe femelle 18 de liaison, par une lumière 17a, longitudinale et débouchante, apte à recevoir une languette.

13a de la largeur réduite de l'élément tractable 13. Cette partie ou languette 13a est de largeur réduite, au moins sur la longueur de la course de chevauchement des extrémités 12a et 12b. La lumière 17a est bordée par deux ailes 17b, dont les extrémités sont coudées en 20 pour former organe de fixation sur le corps des moyens d'actionnement 3.

Le moyen d'actionnement 3 comprend un moteur électrique bidirectionnel 21, qui est logé dans une coque protectrice 22 présentant une partie 23 en matière rigide formant boîtier de moteur, et une partie 24 en matière flexible biocompatible, compressible ou pliable, formant la gaine 14 de la bande flexible 12. La coque 22 peut être, et de préférence est, entièrement réalisée en une seule et même pièce, en matière biocompatible, par exemple, en silicone, la dureté ou rigidité de la matière étant choisie convenablement, pour produire le boîtier rigide et la gaine flexible. Cette coque 22, obtenue par exemple par moulage, permet d'assurer une étanchéité vis-à-vis de fluides biologiques qui pourraient autrement pénétrer dans le boîtier du moteur, et conduire à des dysfonctionnements de ce dernier, ou encore produire des réactions chimiques avec les composants du moteur 21, ayant un effet néfaste pour le patient. Avantageusement, mais de manière non représentée, la gaine 14, présente des évidements de matière, et/ou des plis préformés, de part et d'autre de la bande flexible 12, permettant à la gaine 14 de se plier de manière ordonnée et régulière, lors de l'actionnement de l'organe de constriction 2, et ceci le long et autour du brin de la bande 12, dont la longueur diminue selon la traction de l'extrémité libre 12b de ladite bande 12. Ceci évite également une trop forte contrainte mécanique sur le moteur 21 du moyen d'actionnement 13, et permet de surmonter en même temps la résistance au pliage de l'organe de constriction 2.

Le moteur 21 est lié en rotation à l'organe de traction 25 coopérant avec les crans 15 de l'élément tractable 13. Dans la forme d'exécution des figures 3 et 4, cet organe 25 est une vis tangentielle 15 calée sur l'arbre de sortie 27 de ce moteur 21, et dont le filetage 26 engrène avec les crans 15. Pour réduire l'encombrement général, les axes longitudinaux de la vis 25, de l'arbre 27 et du moteur 21 sont sensiblement dans le plan médian transversal de l'anneau de l'organe de constriction 2. Le moteur 21 est relié électriquement au récepteur intracorporel 11 du moyen de commande 4, par un ou plusieurs fils électriques 28.

Entre le moteur 21 et l'organe de traction 25 est disposé un réducteur (non représenté) avec un rapport de démultiplication important.

Ainsi, le moyen d'actionnement 3 est agencé pour conserver de façon stable la position relative des deux extrémités 12 et 12b de la bande 12, l'une par rapport à l'autre, en l'absence d'apport d'énergie audit moyen d'actionnement 3

Les forces de rappel auxquelles est soumise la bande 12 ne permettent pas d'entraîner en rotation le moteur 21, dont les rotations déterminent le degré de constriction selon l'exemple de réalisation du dispositif de constriction conforme à l'invention.

Lorsque le dispositif de constriction a été implanté dans le corps d'un patient, et la bande flexible 12 fermée dans sa configuration opératoire, par exemple par accouplement des organes de fixation complémentaires 16, 18, la bande présente sensiblement la forme d'un anneau, comme représenté par la Figure 3. Les premiers crans de traction 15, de l'élément tractable 13, sont engrenés avec le filetage 26 de la vis 25 pour maintenir la forme en anneau du dispositif de constriction 2.

La Figure 4 représente la configuration de l'organe de constriction 2, après actionnement du moyen de commande 3, pour réduire le diamètre de la bande flexible 12, et donc augmenter la constriction gastrique. On remarque que la gaine 14, qui est en matière flexible, s'est pliée, ce qui permet à la languette 13a de l'élément tractable 13 de pénétrer dans la lumière 17a de l'extrémité 12a, lors du déplacement qui lui est communiqué par la vis 25.

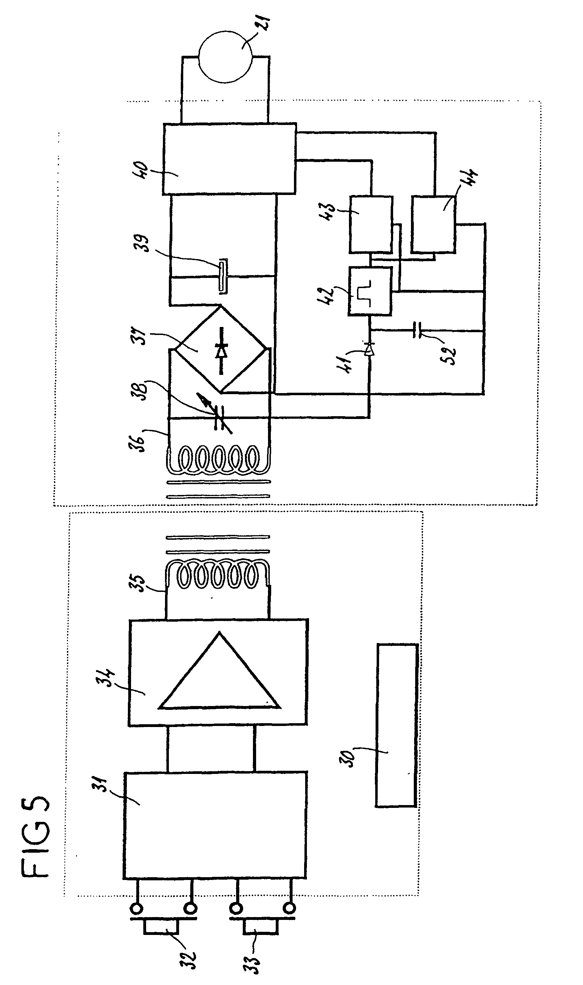

Le moyen de commande 4 sera maintenant décrit en se référant utilement à la Figure 5. Le moyen de commande comprend, dans le mode d'exécution préféré de l'invention, un émetteur 10 extracorporel, et un récepteur 11 intracorporel, par exemple implanté sous la peau, par exemple à la limite inférieure du sternum, ou dans un autre endroit convenable. Le récepteur comporte un moyen de traitement d'un signal émis par l'émetteur 10 et de transmission 29 d'un signal de commande du moyen d'actionnement 3. Ce moyen de traitement et de transmission 29 d'un signal peut également être disposé près du, ou sur, le moyen d'actionnement 3, par exemple sur la coque 22 du moteur 21.

Dans le mode d'exécution préféré, l'émetteur extracorporel 10 est constitué par un circuit électrique comportant une source d'alimentation électrique 30, par exemple une batterie, alimentant un oscillateur 31 à trois fréquences, à savoir une fréquence de chargement F1, une fréquence de marche avant F2, et une fréquence de marche arrière F3. L'oscillateur 31 est relié, d'une part à deux boutons de commande de marche avant 32 et arrière 33 respectivement, et d'autre part à un circuit amplificateur 34. Le circuit amplificateur 34 est relié à une bobine résonante 35, qui présente de préférence un diamètre relativement important par rapport au récepteur 11, par exemple d'environ 30 cm. La bobine résonante 35 peut être une bobine torique d'un fil de cuivre ou d'or, placée dans un anneau de silicone (non représenté), et elle résonne à une fréquence de fonctionnement, correspondant à l'une des trois fréquences précédemment mentionnées, lorsqu'on alimente le circuit depuis la source d'alimentation 30.

Lorsque l'émetteur 10 est mis sous tension, l'oscillateur 31 émet une première fréquence F1 de chargement du circuit, qui est amplifiée par l'amplificateur 34 et envoyée dans la bobine résonante 35, et qui permet de fournir de la puissance au récepteur 11. Celui-ci comporte une bobine d'induction de fréquence 36, de préférence d'environ 3 cm de diamètre, et fonctionnant à une fréquence d'environ 1 MHz. La bobine d'induction 36 est reliée à un pont de diodes 37 et à un condensateur en syntonisation 38, qui règle la fréquence de résonance du circuit récepteur. Le pont de diodes 37 est relié à un condensateur électrochimique 39, permettant de stocker suffisamment d'énergie pour charger et actionner les différents circuits du récepteur, et à un circuit adaptateur 40 d'entraînement du moteur 21. Le signal reçu de la bobine résonante 36 sous forme d'énergie passe par le pont de diodes 37, qui redresse et filtre le signal, et qui fournit ainsi une source d'énergie pour le moteur et les circuits électriques du récepteur. Le condensateur en syntonisation 38 est relié par une diode de détection 41 de signal, à un circuit de mise en forme 42 de la fréquence reçue du condensateur en syntonisation.

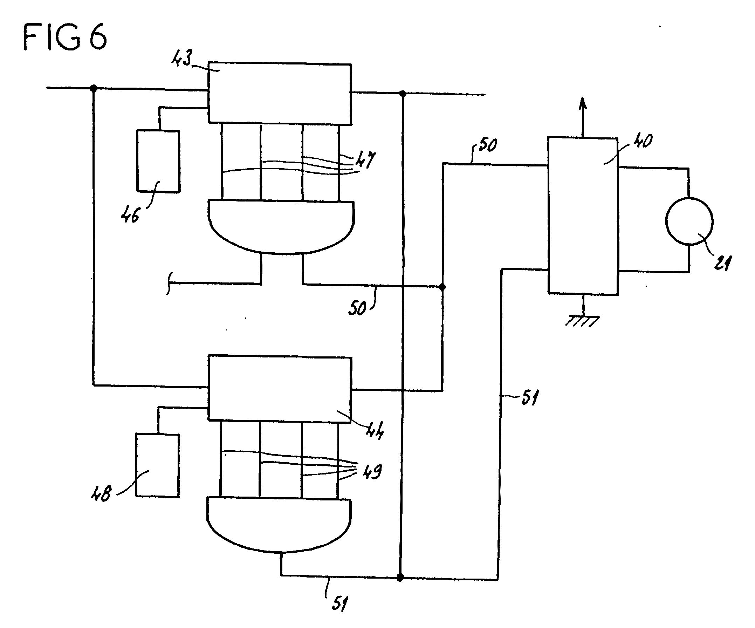

La figure 6 montre schématiquement le circuit de traitement de la fréquence mise en forme et transmis sous forme d'impulsion. Ce signal est transmis à deux circuits de registre à décalage 43, 44, par exemple du type FSK, un premier circuit 43 de registre à décalage étant programmé pour filtrer une fréquence de marche avant F2, et un deuxième circuit 44 de registre à décalage étant programmé pour filtrer une fréquence de marche arrière F3. Le premier circuit 43 de registre à décalage comporte un oscillateur 46, décalant chaque impulsion reçue dans des portes "ET" 47, après un délai prédéterminé calé sur la fréquence de marche avant. Le deuxième circuit 44 de registre à décalage comporte également un oscillateur 48, décalant chaque impulsion reçue dans des portes ET 49, après un délai prédéterminé calé sur la fréquence de marche arrière. Ainsi, ces deux circuits 43, 44 permettent de mesurer la récurrence de l'impulsion fournie par le circuit de mise en forme 42. Les circuits de registre à décalage se terminent par deux sorties 50, 51, reliées à l'adaptateur 40 d'entraînement de moteur, l'une déclenchant l'entraînement dans le sens avant, et l'autre déclenchant l'entraînement dans le sens arrière. Afin d'éviter des interférences de fréquence environnantes, l'émetteur 11 peut également être pourvu d'un condensateur de découplage 52 placé entre le condensateur en syntonisation 38 et le circuit de mise en forme de fréquence 42. Il convient de noter que tous les circuits électriques du récepteur décrits précédemment pourraient également être intégrés dans un circuit microélectronique, ou une puce électronique, et cette puce étant ensuite disposée ou fixée sur le moyen d'actionnement 3.

Le fonctionnement du circuit récepteur sera maintenant décrit par rapport à la description précédente. Si l'opérateur appuie sur le bouton 32 de marche avant de l'émetteur 10, la fréquence F2 est émise, amplifiée, puis induite dans le circuit du récepteur 11. Cette fréquence F2 est détectée par le premier circuit 43 de registre à décalage, comme étant la fréquence de marche avant, moyennant quoi le circuit adaptateur d'entraînement 40 fait tourner le moteur 21, l'arbre 27 et le moyen d'entraînement 25, de manière que ce dernier fasse avancer l'élément tractable 13 en direction du moteur, réduisant ainsi le diamètre de la bande 12. Lorsque l'opérateur appuie sur le bouton de marche arrière 33 de l'émetteur 10, l'effet inverse se produit, et le diamètre de la bande 12 est augmenté.

Ainsi, il est possible de régler de manière très précise, et sans intervention manuelle physique sur les éléments implantés dans le corps, la dimension interne de l'organe de constriction gastrique. Ceci permet un suivi mieux adapté aux besoins du patient à traiter, et des manipulations plus simples à effectuer, car la constriction gastrique appliquée peut être modifiée simplement en approchant l'émetteur 10 devant le récepteur 11 et en l'actionnant.

A titre d'alternative, lorsque le réglage de la constriction est effectué directement par voie percutanée par le chirurgien à l'aide d'instruments appropriés, le moyen d'actionnement 3 ne comporte pas de moteur, et permet d'obtenir un réglage stable et irréversible de la constriction.

## Revendications

1. Dispositif au moins en partie implantable dans le corps humain ou animal, comprenant un organe (2) formant dans sa configuration opératoire un anneau, ledit anneau comportant une bande flexible (12), dont les deux extrémités (12a, 12b) sont adjacentes l'une par rapport à l'autre dans la configuration opératoire, correspondant à la section transversale maximum dudit anneau, ainsi qu'un moyen d'actionnement (3) de l'organe (2) formant anneau, selon lequel, en coopération, d'une part au moins une extrémité (12b) de la bande flexible (12) comporte un élément tractable (13), et d'autre part le moyen d'actionnement (3) comprend un organe de traction (25) de l'élément tractable, permettant de déplacer ladite extrémité (12b) par rapport à l'autre extrémité (12a), en générant une déformation radiale de l'organe (2) formant anneau, **caractérisé en ce que** l'extrémité fixe (12a) de la bande flexible (12) est traversée par une lumière (17a) longitudinale et débouchante, bordée par deux ailes (17b), apte à recevoir de manière chevauchante une languette courbée (13a) de largeur réduite de l'élément tractable (13), dont le mouvement relatif et concentrique par rapport à la bande flexible permet de régler la section transversale, moyennant quoi l'organe formant anneau est un organe de constriction.

2. Dispositif (1) de constriction selon la revendication 1, **caractérisé en ce que** l'organe (2) de constriction comporte une gaine (14) en matière biocompatible, compressible ou pliable, renfermant la bande flexible (12).

3. Dispositif (1) de constriction selon la revendication 1 ou la revendication 2, **caractérisé en ce que** dans une configuration prête à ceindre de l'organe (2) de constriction, entre ses deux extrémités, la bande flexible (12) est composée d'un premier élément (13) comportant un premier organe de fixation (16), par exemple mâle, et d'un deuxième élément (17) comportant un deuxième organe de fixation (18) complémentaire, par exemple femelle, le premier élément (13) étant relié au deuxième élément (17) par accouplement définitif des organes de fixation (16, 18) complémentaires, pour obtenir la configuration opératoire de l'organe (2) de constriction.

4. Dispositif (1) de constriction selon la revendication 2, **caractérisé en ce qu'**il comporte une coque protectrice (22), présentant une partie (23) en matière rigide formant boîtier de moteur (21), et une partie (24) en matière flexible biocompatible compressible ou pliable, formant la gaine (14) de la bande flexible (12).

5. Dispositif (1) de constriction selon la revendication 2 et la revendication 4, **caractérisé en ce que** le boîtier et la gaine (14) forment une seule et même pièce.

6. Dispositif (1) de constriction selon la revendication 2, **caractérisé en ce que** la gaine (14) présente des évidements de matière de part et d'autre de la bande flexible (12), et/ou des plis préformés, permettant à ladite gaine de se plier de manière ordonnée et régulière, lors de la déformation radiale de l'organe de constriction (2).

7. Dispositif (1) de constriction selon l'une quelconque des revendications 1 à 6, **caractérisé en ce qu'**il comprend ou est associé à un moyen de commande (4) à distance.

8. Dispositif (1) de constriction selon l'une quelconque des revendications 1 à 7, **caractérisé en ce que** le moyen de commande (4) comprend un émetteur extracorporel (10) et un récepteur intracorporel (11), ce dernier comportant un moyen (29) de traitement d'un signal émis par l'émetteur (10) d'une part et de transmission d'un signal de commande au moyen d'actionnement (3) d'autre part.

9. Dispositif (1) de constriction selon la revendication 8, **caractérisé en ce que** le moyen de traitement (29) comprend un seul circuit électrique agencé pour véhiculer à la fois des informations de commande et de l'énergie vers le moyen d'actionnement (3).

10. Dispositif (1) de constriction selon la revendication 9, **caractérisé en ce que** le moyen de traitement (29) comprend un circuit électrique constitué par une bobine d'induction résonante (36), un redresseur (37), un générateur d'impulsions (42), au moins un circuit de registre à décalage (43, 44), et un circuit de commande (40) de la direction de marche du moyen d'actionnement (3).

11. Dispositif (1) de constriction selon la revendication 8 ou 9 ou 10, **caractérisé en ce que** le récepteur (11) du moyen de commande (4) est disposé à distance de l'organe (2) de constriction, et relié électriquement (28) au moyen d'actionnement (3).

12. Dispositif (1) de constriction selon l'une quelconque des revendications 8 à 11, **caractérisé en ce que** l'émetteur extracorporel (10) comprend un circuit électrique constitué par une alimentation en énergie électrique (30), un oscillateur (31), un circuit amplificateur (34) et une bobine d'induction primaire (35).

13. Dispositif (1) de constriction selon la revendication 12, **caractérisé en ce que** l'émetteur (10) comporte en outre au moins un organe (32, 33) de sélection de fréquence de l'oscillateur (31).

14. Dispositif (1) de constriction selon l'une quelconque des revendications 1 à 13, **caractérisé en ce que** le moyen d'actionnement (3) est agencé pour conserver de façon stable la position relative des deux extrémités (12a, 12b) de la bande flexible (12), l'une par rapport à l'autre, en l'absence d'apport d'énergie audit moyen d'actionnement (3).

15. Dispositif (1) de constriction selon l'une quelconque des revendications 1 à 13, **caractérisé en ce qu'**il constitue un dispositif de constriction gastrique.

## Patentansprüche

1. Vorrichtung, die zumindest teilweise in den menschlichen oder tierischen Körper implantierbar ist, mit einem Organ (2), das in seinem Betriebszustand einen Ring bildet, wobei der Ring ein flexibles Band (12) aufweist, dessen beiden Enden (12a, 12b) in dem Betriebszustand einander benachbart sind, entsprechend dem maximalen Querschnitt des Rings, sowie mit einem Betätigungsmittel (3) für das den Ring bildenden Organ (2), gemäß dem, im Zusammenwirken, einerseits zumindest ein Ende (12b) des flexiblen Bandes (12) ein zugbetätigbares Element (13) aufweist, und andererseits das Betätigungsmittel (3) ein Zugorgan (25) für das zugbetätigbare Element aufweist, das es ermöglicht, das Ende (12b) bezüglich dem anderen Ende (12a) zu verlagern, wodurch eine radiale Verformung des den Ring bildenden Organs (2) hervorgerufen wird, **dadurch gekennzeichnet, dass** durch das feste Ende (12a) des flexiblen Bandes (12) ein längs verlaufendes und mit Mündung versehenes Loch (17a) verläuft, das durch zwei Schenkel (17b) berandet ist, und das dazu ausgelegt ist, eine gekrümmte Zunge (13a) von verminderter Breite des zugbetätigbaren Elements (13) reitend aufzunehmen, dessen Bewegung relativ und konzentrisch bezüglich des flexiblen Bands es ermöglicht, den Querschnitt einzustellen, wodurch das den Ring bildende Organ ein Organ zum Verengen ist.

2. Vorrichtung (1) zum Verengen nach Anspruch 1, **dadurch gekennzeichnet, dass** das Organ (2) zum Verengen eine Hülle (14) aus biokompatiblem Material aufweist, die kompressibel oder faltbar ist, und die das flexible Band (12) umschließt.

3. Vorrichtung (1) zum Verengen nach Anspruch 1 oder Anspruch 2, **dadurch gekennzeichnet, dass** das flexible Band (12) in einem zum Winden des Organs (2) zum Verengen fertigen Zustand zwischen seinen beiden Enden aus einem ersten Element (13), das ein erstes Befestigungsorgan (16) aufweist, das beispielsweise männlich ist, und aus einem zweiten Element (17) zusammengesetzt ist, das ein komplementäres zweites, beispielsweise weibliches Befestigungsorgan (18) aufweist, wobei das erste Element (13) mit dem zweiten Element (17) durch eine endgültige Kopplung der komplementären Befestigungsorgane (16, 18) verbunden wird, um den Betriebszustand des Organs (2) zum Verengen zu erhalten.

4. Vorrichtung (1) zum Verengen gemäß Anspruch 2, **dadurch gekennzeichnet, dass** sie eine schützende Einhäusung (22) aufweist, die einen Abschnitt (23) aus einem steifen Material, der ein Motorgehäuse (21) bildet, und einen Abschnitt (24) aus kompressiblem oder faltbarem biokompatiblen flexiblen Material aufweist, die die Hülle (14) des flexiblen Bandes (12) bildet.

5. Vorrichtung (1) zum Verengen nach Anspruch 2 und Anspruch 4, **dadurch gekennzeichnet, dass** das Gehäuse und die Hülle (14) ein einziges Stück bilden.

6. Vorrichtung (1) zum Verengen nach Anspruch 2, **dadurch gekennzeichnet, dass** die Hülle (14) Materialaussparungen beidseits des flexiblen Bandes (12) und/oder vorgeformte Falze aufweist, so dass sich die Hülle bei der radialen Verformung des Organs (2) zum Verengen ordnungsgemäß und gleichmäßig falten kann.

7. Vorrichtung (1) zum Verengen nach einem der Ansprüche 1 bis 6, **dadurch gekennzeichnet, dass** sie ein Femsteuermittel (4) aufweist oder einem solchen zugeordnet ist.

8. Vorrichtung (1) zum Verengen nach einem der Ansprüche 1 bis 7, **dadurch gekennzeichnet, dass** das Steuermittel (4) einen extrakorporalen Sender (10) und einen intrakorporalen Empfänger (11) aufweist, wobei Letzterer ein Mittel (29) zur Verarbeitung eines von dem Sender (10) gesendeten Signals einerseits und zur Übertragung eines Steuersignals an das Betätigungsmittel (3) andererseits aufweist.

9. Vorrichtung (1) zum Verengen nach Anspruch 8, **dadurch gekennzeichnet, dass** das Mittel (29) zur Verarbeitung einen einzigen elektrischen Schaltkreis aufweist, der dazu ausgelegt ist, zugleich Steuerinformationen und Energie an das Betätigungsmittel (3) weiterzugeben.

10. Vorrichtung (1) zum Verengen nach Anspruch 9, **dadurch gekennzeichnet, dass** das Mittel (29) zum Verarbeiten einen elektrischen Schaltkreis aufweist, der durch eine Resonanzinduktorspule (36), einen Gleichrichter (37), einen Impulserzeuger (42), einen Schieberegisterkreis (43, 44) und einen Steuerkreis (40) zum Steuern der Richtung des Gangs des Betätigungsmittels (3) gebildet ist.

11. Vorrichtung (1) zum Verengen nach Anspruch 8 oder 9 oder 10, **dadurch gekennzeichnet, dass** der Empfänger (11) des Steuermittels (4) im Abstand von dem Organ (2) zum Verengen angeordnet und elektrisch (28) mit dem Betätigungsmittel (3) verbunden ist.

12. Vorrichtung (1) zum Verengen nach einem der Ansprüche 8 bis 11, **dadurch gekennzeichnet, dass** der extrakorporale Sender (10) einen elektrischen Schaltkreis aufweist, der durch eine elektrische Energieversorgung (30), einen Oszillator (31), einen Verstärkerkreis (34) und eine Primärinduktorspule (35) gebildet ist.

13. Vorrichtung (1) zum Verengen nach Anspruch 12, **dadurch gekennzeichnet, dass** der Sender (10) außerdem zumindest ein Organ (32, 33) zum Wählen der Frequenz des Oszillators (31) aufweist.

14. Vorrichtung (1) zum Verengen nach einem der Ansprüche 1 bis 13, **dadurch gekennzeichnet, dass** das Betätigungsmittel (3) dazu ausgelegt ist, die relative Position der beiden Enden (12a, 12b) des flexiblen Bandes (12) zueinander im Fall der Abwesenheit einer Energiezufuhr zu dem Betätigungsmittel (3) stabil aufrechtzuerhalten.

15. Vorrichtung (1) zum Verengen nach einem der Ansprüche 1 bis 13, **dadurch gekennzeichnet, dass** sie eine Vorrichtung zur Magenverengung darstellt.

## Claims

1. A device which can be at least partially implanted in the human or animal body, comprising a member (2) forming a ring in its operational configuration, said ring including a flexible band (12), of which the two ends (12a, 12b) are adjacent to one another in the operational configuration, corresponding to the maximum transverse section of said ring, and a means (3) for actuating the ring-forming member (2), according to which, in cooperation, on the one hand, at least one end (12b) of the flexible band (12) includes a tractile element (13), and, on the other hand, the actuating means (3) comprises a member (25) for pulling the tractile element, for moving said end (12b) relative to the other end (12a), generating a radial deformation of the ring-forming member (2), **characterized in that** the fixed end (12a) of the flexible band (12) is traversed by a longitudinal and open slit (17a) which is bordered by two wings (17b) and is able to receive, in overlapping manner, a curved tongue (13a), of reduced width, of the tractile element (13), the relative and concentric movement of which relative to the flexible band makes it possible to regulate the transverse section, meaning that the ring-forming member is a constriction member.

2. The constriction device (1) as claimed in claim 1, **characterized in that** the constriction member (2) includes a sheath (14) made of compressible or pliable biocompatible material, enclosing the flexible band (12).

3. The constriction device (1) as claimed in claim 1 or claim 2, **characterized in that**, in a standby configuration of the constriction member (2), between its two ends, the flexible band (12) is made up of a first element (13) including a first fixing member (16), for example male, and a second element (17) including a complementary second fixing member (18), for example female, the first element (13) being connected to the second element (17) by definitive coupling of the complementary fixing members (16, 18), in order to obtain the operational configuration of the constriction member (2).

4. The constriction device (1) as claimed in claim 2, **characterized in that** it includes a protective shell (22) which has a part (23) of rigid material forming a motor housing (21), and a part (24) of compressible or pliable biocompatible flexible material forming the sheath (14) of the flexible band (12).

5. The constriction device (1) as claimed in claim 2 and claim 4, **characterized in that** the casing and the sheath (14) form one and the same piece.

6. The constriction device (1) as claimed in claim 2, **characterized in that** the sheath (14) has material recesses on each side of the flexible band (12), and/or preformed folds, allowing said sheath to fold in an ordered and regular manner upon radial deformation of the constriction member (2).

7. The constriction device (1) as claimed in any one of claims 1 through 6, **characterized in that** it comprises or is associated with a remote control means (4).

8. The constriction device (1) as claimed in any one of claims 1 through 7, **characterized in that** the control means (4) comprises an extracorporeal emitter (10) and an intracorporeal receiver (11), the latter having a means (29) for processing a signal emitted by the emitter (10) and for transmitting a control signal by way of the actuating means (3).

9. The constriction device (1) as claimed in claim 8, **characterized in that** the processing means (29) comprises a single electric circuit designed to carry both control information and power to the actuating means (3).

10. The constriction device (1) as claimed in claim 9, **characterized in that** the processing means (29) comprises an electric circuit consisting of a resonance induction coil (36), a rectifier (37), a pulse generator (42), at least one shift register circuit (43, 44), and a circuit (40) for controlling the direction of running of the actuating means (3).

11. The constriction device (1) as claimed in claim 8 or 9 or 10, **characterized in that** the receiver (11) of the control means (4) is arranged remote from the constriction member (2) and is connected electrically (28) to the actuating means (3).

12. The constriction device (1) as claimed in any one of claims 8 through 11, **characterized in that** the extracorporeal emitter (10) comprises an electric circuit made up of an electrical power supply (30), an oscillator (31), an amplifier circuit (34) and a primary induction coil (35).

13. The constriction device (1) as claimed in claim 12, **characterized in that** the emitter (10) additionally comprises at least one member (32, 33) for selecting the frequency of the oscillator (31).

14. The constriction device (1) as claimed in any one of claims 1 through 13, **characterized in that** the actuating means (3) is designed to retain, in a stable manner, the mutual relative position of the two ends (12a, 12b) of the flexible band (12) in the absence of power supply to said actuating means (3).

15. The constriction device (1) as claimed in any one of claims 1 through 13, **characterized in that** it constitutes a gastric constriction device.
